(19) Europäisches Patentamt

European Patent Office

Office européen des brevets

(11) **EP 0 477 222 B1**

(12) **EUROPEAN PATENT SPECIFICATION**

(45) Date of publication and mention
of the grant of the patent:
**16.08.2000 Bulletin 2000/33**

(21) Application number: **90908952.6**

(22) Date of filing: **13.06.1990**

(51) Int. Cl.[7]: **A61M 15/00**

(86) International application number:
**PCT/FI90/00159**

(87) International publication number:
**WO 90/15635 (27.12.1990 Gazette 1990/29)**

(54) **Device for more effective pulverization of a powdered inhalation medicament**

Vorrichtung zum wirkungsvollen Zerstäuben eines pulverförmigen Medikaments zur Inhalation

Dispositif permettant de réaliser une pulvérisation plus efficace d'un médicament pris par inhalation

(84) Designated Contracting States:
**AT BE CH DE DK ES FR GB IT LI LU NL SE**

(30) Priority: **16.06.1989 FI 892956**

(43) Date of publication of application:
**01.04.1992 Bulletin 1992/14**

(73) Proprietor: **LEIRAS OY**
**20210 Turku (FI)**

(72) Inventor: **LANKINEN, Tapio**
**SF-20800 Turku (FI)**

(74) Representative:
**Palgen, Peter, Dr. Dipl.-Phys. et al**
**König-Palgen-Schumacher-Kluin**
**Patentanwälte**
**Mulvanystrasse 2**
**40239 Düsseldorf (DE)**

(56) References cited:
| | |
|---|---|
| **EP-A- 0 005 585** | **EP-A- 0 215 559** |
| **EP-A- 0 407 028** | **DE-B- 2 449 179** |
| **FI-B- 0 071 488** | **FR-A- 1 445 520** |
| **FR-A- 2 352 556** | **SE-B- 0 453 566** |
| **US-A- 3 809 084** | **US-A- 4 702 415** |

## Description

The present invention relates to a device which is based on centrifugal force for achieving more effective pulverization of a powdered inhalation medicament in a manner that the penetration of medicament into the lungs is improved and the adhesion to the upper respiratory passages is reduced for alleviating the side effects caused thereby.

It is generally known that the size of medicament particles should be 1-5 microns, preferably 2-3 microns, for the best possible penetration into their destination, i.e. deep into the lungs. The most common metering device is a so-called inhalation aerosol which is quite readily capable of reaching the optimal particle size. In addition to inhalation aerosols, an increasing number of powder inhalators are presently in use as these offer certain benefits, e.g. there is no need for ozone-destroying propellants. Several clinical studies have indicated that, with the same amount of medicament, the powder inhalators do not achieve the same effect as inhalation aerosols but it takes up to 2-3 times larger dosages to get the same results. The reason for this is considered to be the fact that a powdered medicament issuing from powder inhalators has too large a particle size. Thus, most of the medicine dosage coming out of inhalators is retained in upper respiratory passages which, with certain medicines, can cause serious side effects. The medicine dosages required for different inhalation medicaments vary considerably, the smallest being appr. 0,01 mg and the largest 20 mg. When small amounts of medicine are metered in powdered form, it is generally necessary to use some adjuvant or carrier, so that the sufficiently precise measuring of a dosage would be possible with the present technology. No matter if the dosage comprises just medicine or has a carrier admixed therein, the medicine dosage substantially comprises inter-adhered particles and most of these agglomerates are too large to penetrate into the lungs. As the agglomerates are released in a powder inhalator into an air flow passing into the lungs of a patient, there will occur some dispersal of these particle deposits, said dispersal resulting from the formulation of a powdered medicament and the construction of an inhalator. It is known that constructions creating a strong turbulence are capable of more effective pulverization.

In practice, however, no prior known powder inhalator structure and/or medicine formulation has produced results that would be equal to those achieved by an ordinary inhalation aerosol. It has been suggested as a partial solution that inhalation should be effected with as much force as possible, whereby the turbulence and pulverization of particles would accordingly be most effective. However, a quick inhalation is difficult for a person suffering e.g. from serious asthma and, on the other hand, a quick inhalation increases the residue in upper respiratory tracts. According to studies, pulverization of agglomerates is indeed intensified but the overall benefit is marginal. The best pulmonary penetration in relation to the adherence of medicament to upper respiratory tracts has been achieved by slow inhalation, corresponding to a flow rate of appr. 30 l/mm or 0,5 l/sec.

The only prior known powder inhalator is the device described in Finnish Patent application No. 871000 which has been designed in an effort to produce a clearly defined turbulence for pulverizing agglomerations of medicine. The centrally directed deflectors inside the device or the helical chute are explained to set the air flow in a spinning motion, whereby the medicine particles entrapped in the air abrade as a result of centrifugal force against the walls of the structure as well as collide into each other with resulting pulverization. The device described in the cited application has been marketed under a tradename Turbuhaler® (Draco, Sweden) and the pulverizing structure therein is a helical chute or groove. Laboratory tests indicated that this device had a relatively good pulverization of agglomerates of medicine which could be very distinctively intensified, however, by means of a device of the present invention. In view of the pulverization of agglomerates or accumulations of medicine, there are a few defects in the device. The helical groove has in the centre thereof an open space having less air resistance than inside the groove. Accordingly, the flow rate of air and centrifugal force on the circumference of the groove are less than theoretical. Since the particles advance in the groove under a force caused by air resistance and centrifugal force tends to push the particles perpendicularly to the circumferential tangent, the actual force applied to the particles is a resultant of these forces and is applied diagonally relative to the circumferential tangent. Thus, the centrifugal force resulting from the spinning motion cannot be utilized in full extent for the pulverization of accumulations. In all deflector structures according to the cited application, the particles escape from the device within a few thousandths of a second when using conventional inhalation rates of 30-60 l/min and that is a very short time for an effective pulverization. The residence time can be lengthened e.g. by increasing the number of helices in groove portions or the number of separate deflector structures or the length of zigzagging air flow channels, but this would complicate manufacturing and cleaning and medicine residues in the actual device would increase. After all, cleaning of the structures disclosed in the cited application is difficult as it is.

The European Patent application No. 215559 discloses a powder inhalator, wherein one or more balls travel as a result of air flow around a periphery which is substantially circular in configuration. The air flow comes into contact with the periphery tangentially relative thereto. The medicine is adhered either to the surface of balls or to the surface of the circulation periphery from which it is removed and is pulverized by the action of the rolling balls. The device employs a centrifugal force for fractionating loose particles in a manner that the discharge of air occurs centrally relative to the circulating path. Thus, the pulverization of medicine is a result of a mechanical contact between the balls and the surface.

In the cited structure, the balls close the circulating path for the most part and, thus, there cannot be high speeds of circulation for the balls or medicine particles and, hence, there cannot be major centrifugal forces. It is obviously difficult to use the device for repeatedly metering out exact doses of medicine.

The British Patent No. 1485163 describes a device, wherein a powdered medicament containing elongated capsule provided with pierced ends is set through the action of inhalation air in a rotating motion inside a cylindrical mixing chamber. Piercing of the capsule is effected in a capsule-shaped space which is in open communication with the mixing chamber and the capsule is jerked therefrom along with the air flow into the mixing chamber to spin around its vertical axis. The medicament flings through the ends of the capsule into the mixing chamber and further into an inhalation channel. The device according to this Patent has been marketed under the tradename Inalatore I.S.F. Laboratory tests showed that the device had a reasonable pulverizing effect for accumulations of medicine but a distinctly poorer effect than what is achieved by a device of the present invention.

The device disclosed in the cited Patent would have an improved pulverizing effect if the rotating speed of a capsule and air in the mixing chamber could be increased for using the centrifugal force more effectively for pulverization. This is impossible with the cited structure since it is prevented by the own mass of a capsule and by the friction resulting from its rotation. In addition, the space in communication with the mixing chamber and intended for piercing the capsule is asymmetrical relative to rotating direction and produces a decelerating turbulence.

The British Patent No. 1331216 of the same Patent Owner discloses a device operating on the capsule discharging mechanism, wherein the capsule after piercing is carried into a cylindrical mixing chamber by the action of inhalation. The air arrives in this chamber through a plurality of tubes directed tangentially to the circulation periphery setting the capsule in a rotating motion and transferring the medicine from the capsule into the inhalation air. This structure is also not capable of producing sufficient centrifugal forces for the pulverization of accumulations of medicine because of the capsule's mass, rotational friction and air resistance.

The British Patent No. 1472650 discloses a device for the inhalation of a powdered medicament contained in a capsule. The capsule is purged in a manner that some of the inhalation air is passed through a pierced capsule while most of the air travels past the capsule. However, piercing of the capsule is effected centrally towards the longitudinal axis of the capsule and there is no purpose to create inside the capsule a turbulent flow that would produce a major centrifugal force. Also, according to laboratory tests, the device set forth in the cited Patent (Boehringer Ingelheim) did not produce a powerful turbulence inside the capsule. Also the pulverizing effect of the device for accumulations of medicine was conventional.

The British Patent No. 1118341 describes a structure for purging an open, medicine-containing container into inhalation air. As one alternative to sucking the air into a chamber containing a medicine container there is shown a structure which uses deflectors for setting the air flow in a spinning motion in the chamber. The. cited Patent specification discloses that an object is rather to create irregular turbulence and passage of air flows against the deflectors than to set the air in a rotating motion as rapid as possible. Thus, the internal positioning of deflectors in the chamber severely restricts the rotating motion but creates effectively other turbulence.

Prior known are also several structures, wherein a medicament- containing capsule is pierced prior to dosage, set in its holder in a rotating motion by means of inhalation air or cut open. Prior known are also structures, wherein a medicament is transferred from a capsule into inhalation air by the application of pressurized air. Furthermore, there are known structures, wherein a powdered medicament is transferred for inhalation from a disc or a separate powdered medicament container carrying several doses of medicine. US 40 46 146, 41 16 195, 41 17 844, 42 10 140, GB 11 82 779, 13 96 258, 14 04 338, 14 57 352, 14 59 426, 15 02 150, 15 21 000, Finnish Patent Publication 76 256, Finnish application 863 094 and 883 767, Danish Publication Print 153 631 B.

EP-A-005 585 discloses an inhalation device in which the opening of the capsule initially containing the medicament is achieved simply in situ. The device is provided with a swirl chamber, depression means in said chamber for locating the capsule in a position to be opened and piercing opening means. Air entering the swirl chamber disloads the capsule from the depression and causes it to rotate about its axis. This movement causes the medicament to escape through the holes pierced in the capsule and become entrained in the air stream to be carried into the mouth. The capsule is contrained within the swirl chamber by a sieve.

The DE-A-24 49 179 discloses an inhalation device for a powdered composition that facilitates the emptying of a capsule. The device is provided with a circular swirl chamber and a cavity for the capsule below said chamber. The cavity is provided with piercing means for perforation of the capsule. The pierced capsule is ejected into the swirl chamber and will be set in a propeller-like movement inside said chamber.

None of the above cited and examined publications discloses a structure, wherein a powdered medicament would be pulverized by means of inhalation by the application of a centrifugal force resulting primarily from a powerful rotating motion with a structure described hereinafter.

EP-407 028 A2 is not prepublished, but has the better priority. It shows in figs. 5 to 7 a vortex chamber, into which an inlet tangentially directs air with pulverized medicament. The dispersion air/medicament leaves the chamber axially at an outlet. Nothing is said about the diameter of the chamber.

The preamble of the new main claim is based on FR-A-23 52 556 showing a cylindrical vortex chamber closed at one end, operated by the action of inhalation and having one tangential air-medicament inlet duct, an additional tagential air inlet duct and an axial outlet duct near the same end of the chamber as the inlet duct. The outlet is formed by a tube-like connection extending beyond the zone of the inlet duct and impeding the air flow. Nothing is said again about the diameter of the cylindrical chamber.

It is the object of the invention to obtain an optimal pulverization of agglomerates of a medicament to be inhaled.

The object is achieved by virtue of a device having the features recited in claim 1.

Further advantageous features of the invention are subject matter of the subclaims.

In a device of the invention according to claim 1 a powdered medicament intended for inhalation is pulverized on the basis of a sufficiently powerful centrifugal force prior to or during inhalation. The centrifugal force is produced through the action of inhalation. In a device of the invention, a powdered medicament is entrapped in a gas flow and forced in a substantially circular or rotationally symmetrical space to such a powerful rotating motion that an effective splitting of accumulations of medicine is obtained. This is effected in a rotationally symmetrical chamber whose largest internal diameter cna be 20 millimeteres. With a device of the invention, the pulverization time of large, hard-splitting particles can be increased and, as the rotating motion is over, the mayor particles, e.g. the carrier, can be mostly retained in the chanter to prevent its passage into the repiratory tracts of a patient. A device of the invention is distinctly more effective than the prior known solutions, and thus, as well as by virtue of the ability of retaining large particles, it is possible to improve the effect of medication and to reduce the side effects caused by a medicament remaining in the upper respiratory tracts.

Upon the application of this device to inhalation conducted by a patient, it should be appreciated that the best penetration of medicine particles into the lungs is obtained by means of a slow inhalation with a duration of appr. 5 seconds. Thus, the inhalation rate will be 20-30 l/min. In order to facilitate such trouble-free operation e.g. for a person with a difficult asthma, the inhalation resistance caused by the device itself may not be too high. However, all deviations from a laminar air flow add to the inhalation resistance in powder inhalators no matter how effectively such deviation or turbulence pulverizes accumulations of medicine. Because, however, the inhalating power of a patient sets a practical limit to the force that can be used in an inhalator for the pulverization of accumulations of medicine, the optimal exploitation of this force is of major importance in view of the proper operation of the device. When developing this device, various turbulences and collision patterns of particles were compared with centrifugal force and that latter was found overwhelmingly superior.

If a cylinder with one solid end is supplied with an air flow tangentially from the side at its solid end, such flow is first set in a rotating motion dictated by its entrance speed which produces a centrifugal force. Magnitude of this force can be calculated from the formula:

$$a = -\frac{v^2}{r}$$

wherein

a = acceleration
v = air flow rate
r = radius of cylinder

When gravity acts on a mass at an acceleration of 9,91 m/s$^2$, the a: 9,91 m/s$^2$ indicates the number of times the mass (weight) of a particle circulating along the inner wall of a cylinder entrapped in an air flow is multiplied as a result of the centrifugal force.

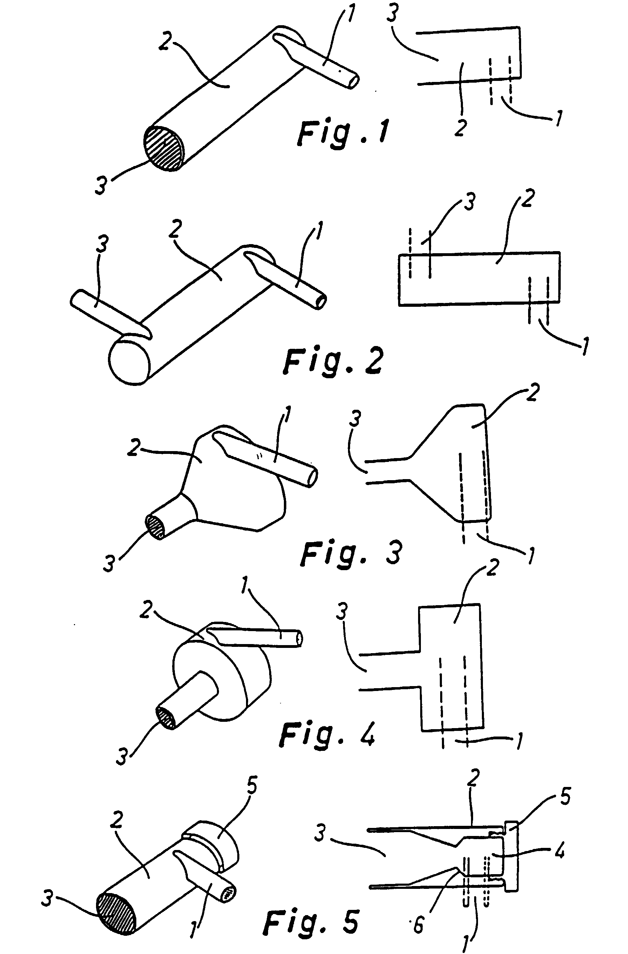

If in such a well-operating device (fig. 1) the radius of an inlet tube is 3 mm and the radius of a vortex cylinder is 6 mm, the suction rate of 30 1/min corresponding to a slow inhalation provides a maximal air circulation rate of 17,68 m/s in the cylinder and an acceleration of 52,1 x 10$^3$ m/s$^2$, the latter being 5310 times the acceleration of gravity. According to this, the weight of medicine particles would be multiplied by more than 5000, which fully expalins the power of the device. When measuring the negative pressure caused by inhalation at a suction rate of 0,5 l/s, the discovered reading was -15 mbar but when inhaling in the reverse direction, the reading was just -4,5 mbar. The difference reflects the energy required for the generation of a centrifugal force since, when inhalating in the reverse direction, there will be no turbulent flow and air resistance is quite close to that of a laminar flow. Tests on patients have revealed that the inhalation resistance should not exceed the reading corresponding to a negative pressure of 15-20 mbar. On the other hand, a suitable inhalation resistance can be used to prevent too fast an inhalation as the latter would increase the medicine residue in upper respiratory tracts. Hence, in a device of the invention it is possible to set an inhalation resistance particularly by adjusting the diameter of an inlet tube and that of the cylinder while the force of inhalation can still be effectively used for the pulverization of accumulations of medicine.

Because of the operating principle of the device, the cylinder cannot be allowed to contain any structures substantially impeding free air circulation, such as deflectors, grooves or capsules or parts thereof spinning along with the air flow, with the exception of carriers containing medicine particles or a formulation. Even the relatively large amounts of carriers contained in certain medical formulations clearly hamper and decrease the speed of rotation. The cylinder must have a cross-section which is substantially circular in every part thereof. However, this makes it possible that the cylinder can have a cross-section which is e.g. conical or symmetrically multiformed for using a centrifugal force for the fractionation of particles by the application of generally known centrifugating principles. A rotationally symmetrical axle or a part thereof extending in the same direction as the longitudinal axis of a vortex chamber does not disturb the action.

The following are examples of devices of the invention. Fig. 1 shows a cylindrical device having a cross-section which is in all aspects in the form of an equiradius circle. Fig. 2 shows a cylinder which is solid at both ends and both the entrance and exit of air occur tangentially. In fig. 3 the cylinder has a conical cross-section and in fig. 4 a quadratic cross-section, the discharge being effected centrally through the gable of the cylinder. In these structures, during a circulating motion, there occurs fractionation of particles in a manner that larger particles tend to circulate continuously on the largest periphery of the cylinder and shall not be able to escape through the central outlet port until pulverized to sufficient fineness.

It has been found out experimentally that the pulverization time of large particles can be further increased if the structure of fig. 3 or 4 is alongside the inlet tube provided with a solid chamber extension. At the end of air flow, the non-pulverized particles are mostly retained in this space and cannot work their way into the pharynx of a patient. Fig. 5 shows a more detailed structural drawing of such a vortex chamber. The medicine agglomerates arrive along with an air flow from a tube 1 into a chamber provided with a constriction 6 for preventing the immediate departure of large particles from the chamber under the action of a centrifugal force. The large particles are able to rotate and spin in a closed chamber section 3 and, after a sufficient pulverization, are able to escape into an inhalation tube 4. The closed chamber section comprises a removable plug 5 for facilitating the cleaning of the chamber. The optimum diameter of a vortex chamber operating by the action of inhalation is 10-20 mm. The pulverization effect is excellent and the substantially tangential setting of an inlet tube is possible as long as the air resistance remains reasonable. If the diameter is increased, the pulverization effect deteriorates in a manner that, with a diameter of more than 30 mm, the pulverization effect is no longer significant.

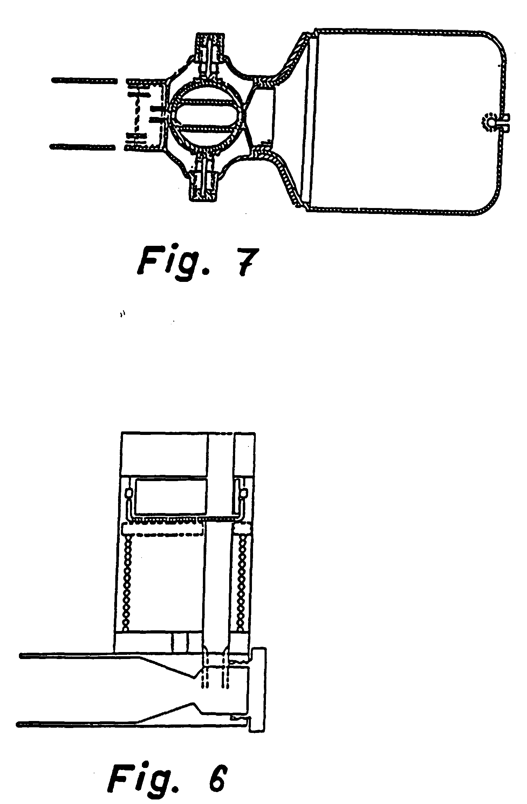

The devices shown in figs. 1-5 can be connected to all available powder inhalators. In fig. 6, a device as shown in fig. 5 is connected to a powder inhalator (Turbuhaler®, Draco, Sweden) described in Finnish Patent Application No. 871000 in a manner that the device replaces the helical groove included in Turbuhaler®.

In fig. 7, a version of the device shown in fig. 2 fitted with two outlet tubes is connected to a powder inhalator described in Finnish Patent Application No. 883767, wherein a medicine capsule is emptied by means of compressed air produced with a hand pumpet. In this type of combination, the inhalation must be effected at the same time as the pumpet is pressed. Inhalation air is picked up from the area alongside the vortex chamber outlet tubes.

The operating ability of a device of the invention is highly dependent on the properties of a presently used medicament and possible additives. In order to achieve the best possible result, different medical formulations require the use of different vortex chamber designs. The manufacturing material of a vortex chamber must also be selcted in a manner that the adherence of a medicament to the chamber is as insignificant as possible and that the chamber has an inner surface which withstands major abrasive forces without excessive wear.

The power of a device of the invention ahs been studied by the application of a method generally used in this field, wherein the inhalation effected by a patient is simulated to such a powdered medicament into a particle separator (a cascade impactor). This is to find out the number and mean particle size of thos medicine particles that are capable of passing into their pulmonary site of action (less than 5,8 microns).

The following table illustrates results of the outputs of a device of the invention as well as prior known powder inhalators included as a reference.

| Powdered medicine | | Inhalator | % of particles less than 5,8 microns of a dosage | Mean particle size (micron) | Patent reference to inhalator |
|---|---|---|---|---|---|
| 1.1 | Ventoline 0,2 mg Rotacaps | Rotahaler® | 22,7 | 7,6 | Danish publ. No. 153631 B |

(continued)

| Powdered medicine | | Inhalator | % of particles less than 5,8 microns of a dosage | Mean particle size (micron) | Patent reference to inhalator |
|---|---|---|---|---|---|
| 1.2 | Ventoline 0,2 mg Rotacaps | Inalatore I.S.F. | 30,3 | 5,8 | GB 1485163 |
| 1.3 | Ventodisks 0,2 mg | Diskhaler® | 26,0 | 5,8 | Finnish Pat.appl. No. 863094 |
| 1.4 | Ventoline 0,2 mg Rotacaps | Fig. 5 Prototype | 61,6 | 2,4 | |
| 2.1 | Lomudal 20 mg caps. | Spinnhaler® | 14,0 | 9 | GB 1182779 |
| 2.2 | Lomudal 20 mg caps. | Fig. 5 Prototype | 38,2 | 2,3 | |
| 3.1 | Bricanyl 0,5 mg | Turbyhaler® | 35,5 | 4,1 | Finnish Pat.appl. No. 871000 |
| 3.2 | Bricanyl 0,5 mg | Fig. 4 Prototype | 58,3 | 2,4 | |

In order to obtain comparable results, all reference groups employed the same pharmaceutical formulations:

1.1 - 1.4    salbutamol as pharmaceutical, lactose as carrier (Glaxo, GB)
2.1 - 2.2    Na-chromokligate as pharmaceutical, no carrier (Fisons, GB)
3.1 - 3.2    terbutaline as pharmaceutical, no carrier (Draco, S)

In prototypes of the invention, the dosage of a powdered medicine was effected by means of the metering unit of a powder inhalator according to US Patent 4046146 which, if used by itself, does not have a distinct particles pulverizing effect as the same results were obtained by a manual powder feeding. Other inhalators included in the comparison are commercially available. The results take into consideration also the medicament stuck in the metering unit and inhalators.

When assessing the results, an objective of powder inhalators should also be considered: to administer as much as possible of a medicine dose into the inhalation of a patient as particles whose size is 1-5 microns, preferably 2-3 microns, for the most likely pulmonatory penetration.

In all reference groups, a prototype of the invention was overwhelmingly the best. The number of pharmaceutical particles of the proper size category was 1,6 - 2,7 times more than that of reference particles and the particles had exactly the optimum mean size.

Thus, a device of the invention is capable of considerably improving the penetration of a medicine into the lungs and, thus, to reduce the residue in upper respiratory tracts for alleviating the side effects caused thereby. The present structures are readily cleanable e.g. with a small brush. The structures can be readily manufactured e.g. as pressure casting of plastics. A device of the invention can be connected to all prior known powder inhalators. It can be used both with separate medicine capsules and in association with a powder container containing a plurality of doses.

**Claims**

1. A device for an effective pulverization of agglomerates of a powdered inhalation medicament, the device comprising a vortex chamber (2) operated by the action of inhalation, the vortex chamber (2) having a center axis and being closed at least at one end thereof, the cross-section of said vortex chamber (2) perpendicular to the center axis thereof being substantially circular in shape, the vortex chamber (2) being provided with at least one air inlet (1) directing the flow of air into the vortex chamber (2) tangentially in a plane perpendicular to said center axis, and an outlet (3) for directing the air and the medicament particles contained therein into the airways of the user of the device, the air inlet (1) being adjacent to the closed end of the vortex chamber (2) and the outlet (3) being spaced from the inlet (1) in the direction of the center axis at the other end of the vortex chamber (2), and the vortex chamber (2) not containing any flow obstacles and having a diameter between 10 to 20 millimeters, and a metering unit connected to the vortex chamber (2) or to the air inlet (1) effecting a dosage of a powdered medicine into the vortex chamber or into the air flow entering the vortex chamber.

2. A device as set forth in claim 1, **characterized in** that the vortex chamber (2) is cylindrical.

3. A device as set forth in claim 1, **characterised in** that the diameter of vortex chamber (2) varies in a stepless and/or stepwise fashion.

4. A device as set forth in claim 3, **characterized in** that the vortex chamber (2) is funnel-like or conically shaped.

5. A device as set forth in anyone of claims 1 to 4, **characterized in** that said vortex chamber (2) is closed at both ends thereof.

6. A device as set forth in any of claims 1 to 5, **characterized in** that between vortex chamber (2) and outlet (3) is fitted a constriction (6) for retaining mayor particles.

7. A device as set forth in claim 1, 2 or 3, **characterized in** that the closed end of vortex chamber (2) is a rotationally symmetrical space (4) which consists of a removable plug (5).

8. A device as set forth in any of claims 1 to 7, **characterized in** that the vortex chamber is made of a material with insignificant adherance to the medicament.

9. A device as set forth in any of claims 1 to 8, **characterized in** that the inner surface of the vortex chamber is made of a material which withstands major abrasive forces without excessive wear.

**Patentansprüche**

1. Vorrichtung zur effektiven Zerstäubung von Zusammenhäufungen eines pulverförmigen Inhalationsmedikamentes, wobei die Vorrichtung eine Vortexkammer (2), die durch die Wirkung der Inhalation betrieben wird, wobei die Vortexkammer (2) eine mittlere Achse besitzt und an wenigstens einem Ende geschlossen ist, wobei der Querschnitt der Vortexkammer (2) senkrecht zu deren Mittelachse im wesentlichen kreisförmiger Gestalt ist, die Vortexkammer (2) mit wenigstens einem Lufteinlaß (1) versehen ist, der die Strömung der Luft tangential in eine senkrecht zu der Mittelachse stehende Ebene in die Vortexkammer (2) leitet und einen Auslaß (3) zur Leitung der Luft und der darin enthaltenen Medikamentpartikel in die Atemwege des Benutzers der Vorrichtung, wobei der Lufteinlaß (1) dem geschlossenen Ende der Vortexkammer (2) benachbart ist und der Auslaß (3) von dem Einlaß (1) in Richtung der Mittelachse beabstandet an dem anderen Ende der Vortexkammer (2) angeordnet ist und die Vortexkammer (2) keine Strömungshindernisse beinhaltet und einen Durchmesser zwischen 10 und 20 mm besitzt, und eine mit der Vortexkammer (2) oder dem Lufteinlaß (1) verbundenen Abmeßeinheit zur Abgabe einer Dosis eines gepulverten Medikaments in die Vortexkammer oder in eine in die Vortexkammer eintretende Luftströmung umfaßt.

2. Vorrichtung nach Anspruch 1, dadurch gekennzeichnet, daß die Vortexkammer (2) zylindrisch ist.

3. Vorrichtung nach Anspruch 1, dadurch gekennzeichnet, daß der Durchmesser der Vortexkammer (2) stufenlos und/oder stufenweise variiert.

4. Vorrichtung nach Anspruch 3, dadurch gekennzeichnet, daß die Vortexkammer (2) trichterförmig oder konisch ausgebildet ist.

5. Vorrichtung nach irgendeinem der Ansprüche 1 bis 4, dadurch gekennzeichnet, daß die Vortexkammer (2) an beiden Enden geschlossen ist.

6. Vorrichtung nach irgendeinem der Ansprüche 1 bis 5, dadurch gekennzeichnet, daß zwischen der Vortexkammer (2) und dem Auslaß (3) eine Verengung (6) zur Zurückhalten großer Partikel angebracht ist.

7. Vorrichtung nach Anspruch 1, 2 oder 3, dadurch gekennzeichnet, daß das abgeschlossene Ende der Vortexkammer (2) ein rotationssymmetrischer Raum (4) ist, der aus einem entfernbaren Stopfen besteht.

8. Vorrichtung aus irgendeinem der Ansprüche 1 bis 7, dadurch gekennzeichnet, daß die Vortexkammer aus einem Material hergestellt ist, welches eine unbedeutende Haftung zu dem Medikament aufweist.

9. Vorrichtung nach irgendeinem der Ansprüche 1 bis 8, dadurch gekennzeichnet, daß die innere Oberfläche der Vortexkammer aus einem Material hergestellt ist, welche große Abriebkräften ohne übermäßige Abnutzung widersteht.

**Revendications**

1. Dispositif en vue d'une pulvérisation efficace d'agglomérés d'un médicament d'inhalation en poudre, le dispositif comprenant une chambre à vortex (2) actionné par l'action de l'inhalation, la chambre à vortex (2) présentant un axe central et étant fermée au moins à une de ses extrémités, la section transversale de ladite chambre à vortex (2), perpendiculaire à son axe central, étant de forme essentiellement circulaire, la chambre à vortex (2) étant dotée d'au moins une entrée d'air (1) dirigeant l'écoulement d'air dans la chambre à vortex (2) tangentiellement dans un plan perpendiculaire au dit axe central, et une sortie (3) pour diriger l'air et les particules de médicament qui y sont contenues dans les voies aériennes de l'utilisateur du dispositif, l'entrée d'air (1) étant adjacente à l'extrémité fermée de la chambre à vortex (2) et la sortie (3) étant écartée de l'entrée (1) à l'autre extrémité de la chambre à vortex (2) dans la direction de l'axe central, et la chambre à vortex (2) ne contient aucun obstacle à l'écoulement et présente un diamètre compris entre 10 et 20 mm, et une unité de dosage connectée à la chambre à vortex (2) ou à l'entrée d'air (1) réalisant un dosage d'un médicament en poudre dans la chambre à vortex ou dans l'écoulement d'air pénétrant dans la chambre à vortex.

2. Dispositif selon la revendication 1, caractérisé en ce que la chambre à vortex (2) est cylindrique.

3. Dispositif selon la revendication 1, caractérisé en ce que le diamètre de la chambre à vortex (2) varie sans discontinuité et/ou par pas.

4. Dispositif selon la revendication 3, caractérisé en ce que la chambre à vortex (2) est configurée en entonnoir ou en cône.

5. Dispositif selon l'une quelconque des revendications 1 à 4, caractérisé en ce que ladite chambre à vortex (2) est fermée à ses deux extrémités.

6. Dispositif selon l'une quelconque des revendications 1 à 5, caractérisé en ce qu'entre la chambre à vortex (2) et la sortie (3) est prévu un rétrécissement (6) servant à retenir les plus grandes particules.

7. Dispositif selon la revendication 1, 2 ou 3, caractérisé en ce que l'extrémité fermée de la chambre à vortex (2) est un espace (4) à symétrie de rotation qui est constitué d'un bouchon (5) amovible.

8. Dispositif selon l'une quelconque des revendications 1 à 7, caractérisé en ce que la chambre à vortex est réalisée en un matériau qui présente une adhérence très faible au médicament.

9. Dispositif selon l'une quelconque des revendications 1 à 8, caractérisé en ce que la surface intérieure de la chambre à vortex est constituée d'un matériau qui résiste aux importantes forces d'abrasion sans usure excessive.

Fig. 1

Fig. 2

Fig. 3

Fig. 4

Fig. 5

*Fig. 7*

*Fig. 6*